Europäisches Patentamt

European Patent Office

Office européen des brevets

(19)

(11) Veröffentlichungsnummer: **0 011 847**

**A1**

(12) # EUROPÄISCHE PATENTANMELDUNG

(21) Anmeldenummer: **79104697.2**

(22) Anmeldetag: **26.11.79**

(51) Int. Cl.³: **A 62 B 9/00**
**A 61 M 16/00**

(30) Priorität: **29.11.78 DE 2851564**

(43) Veröffentlichungstag der Anmeldung:
**11.06.80 Patentblatt 80/12**

(84) Benannte Vertragsstaaten:
**FR GB IT NL SE**

(71) Anmelder: **SIEMENS AKTIENGESELLSCHAFT Berlin
und München
Postfach 22 02 61
D-8000 München 22(DE)**

(72) Erfinder: **Gedeon, Andras
Burschstigen 3A
S-18330 Täby(SE)**

(72) Erfinder: **Psaros, Georgios
Adler salvius väg 122
S-14600 Tullinge(SE)**

(72) Erfinder: **Olsson, Sven-Gunnar
Malmvägen 47C
S-19161 Sollentuna(SE)**

(54) **Vorrichtung zum Anwärmen und Befeuchten eines Beatmungsgases.**

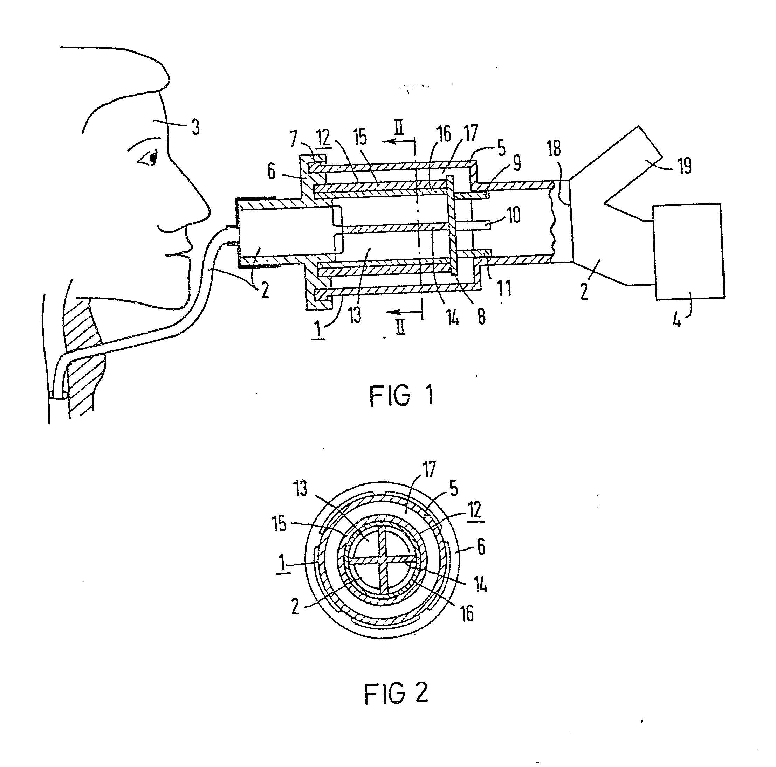

(57) Die Erfindung betrifft eine Vorrichtung zum Anwärmen und Befeuchten eines Beatmungsgases für einen Patienten (3). Die Vorrichtung, die in einem Gehäuse (5), welches in einer zum Patienten (3) und zu einem Beatmunsgasbehälter (4) führenden Gasleitung (2) angeordnet ist, enthält einen Feuchtigkeitsspeicher (12) aus gasdurchlässigem, hygroskopischem Material. In dem Gehäuse (5) ist ein mit der zum Patienten (3) führenden Gasleitung (2) verbundener Hohlraum (13) vorgesehen, der von einem Hohlzylinder (12) aus dem hygroskopischen Material umschlossen ist (Fig. 1).

FIG 1

EP 0 011 847 A1

Croydon Printing Company Ltd.

0011847

SIEMENS AKTIENGESELLSCHAFT          Unser Zeichen
Berlin und München                  VPA 78 P 5951 EUR


Vorrichtung zum Anwärmen und Befeuchten eines
Beatmungsgases

Die Erfindung betrifft eine Vorrichtung zum Anwärmen
und Befeuchten eines Beatmungsgases für einen Patienten, die in einem Gehäuse, welches in einer zum Patienten und zu einem Beatmungsgasbehälter führenden Gasleitung angeordnet ist, einen Feuchtigkeitsspeicher
aus gasdurchlässigem, hygroskopischem Material enthält.

Es ist durch den Prospekt "Portex Luftspeicher" der
Fa. Gibecks, Schweden, eine Vorrichtung dieser Art bekannt, bei der der Feuchtigkeitsspeicher aus einem gerollten Papierstreifen besteht, wobei die Längsachse
der Rolle in Strömungsrichtung des Atemgases angeordnet
ist. Die Feuchtigkeit und die Wärme des von dem Patienten ausgeatmeten Gases wird in den Luftspalten zwischen
den Papierschichten der Rolle gespeichert. Ferner saugt
das Papier einen Teil der Feuchtigkeit auf und speichert
auch einen Teil der Wärme. Beim Einatmen des Patienten

Lst 5 Kli / 27.11.1978

wird das Einatmungsgas bei Passieren der Rolle angewärmt und befeuchtet. Diese Vorrichtung vermag nicht
genug Wärme und Feuchtigkeit von dem Ausatmungsgas
zu speichern, um sehr trockenes Beatmungsgas, z.B.
bei Respiratorbehandlungen, ausreichend anzuwärmen
und zu befeuchten.

Es ist ferner durch den Prospekt "Cascade Humidifer"
der Fa. Bennet, USA, eine Vorrichtung zum Anwärmen
und Befeuchten eines einem Patienten zugeführten
trockenen Beatmungsgases bekannt, die einen mit warmem
Wasser gefüllten Behälter aufweist, bei der das Einatmungsgas durch das Wasser geleitet wird. Dadurch
wird das Gas angewärmt und erhält eine hohe Luftfeuchtigkeit. Diese Vorrichtung muß wegen ihres Aufbaus verhältnismäßig weit entfernt vom Patienten angeordnet werden, so daß sich das Beatmungsgas in der Verbindungsleitung zum Patienten wieder abkühlen kann.
Ferner kann sich dort Kondenswasser bilden. Um dies
zu vermeiden, kann die Verbindungsleitung in Abhängigkeit von der am Mund des Patienten gemessenen Temperatur des Atemgases angewärmt werden. Dies ergibt eine
sperrige, teuere und im Aufbau komplizierte Ausbildung.

Es ist weiterhin durch die schwedische Patentanmeldung 76 033 968 eine Vorrichtung zum Anwärmen und Befeuchten eines einem Patienten zugeführten trockenen
Beatmungsgases bekannt, die in einem Gehäuse rechtwinkelig zur Strömungsrichtung des Beatmungsgases
mehrere Scheiben aufweist, von denen jeweils eine
feuchtigkeitsabsorbierend sowie wärmeisolierend und
die benachbarte wärmeleitend ist. Bei dieser Vorrichtung können die wärmeleitenden Scheiben wegen ihrer
geringen Oberfläche nicht in ausreichendem Maße Wärme
speichern.

- 3 -       VPA 78 P 5951 EUR

Der Erfindung liegt die Aufgabe zugrunde, eine Vorrichtung der eingangs genannten Art zu schaffen, die im Aufbau einfach ist und bei der eine ausreichende Aufwärmung und Befeuchtung auch eines sehr trockenen Einatmungsgases gewährleistet ist.

Diese Aufgabe ist erfindungsgemäß dadurch gelöst, daß in dem Gehäuse ein mit der zum Patienten führenden Gasleitung verbundener Hohlraum vorgesehen ist, der von einem Hohlzylinder aus dem hygroskopischen Material umschlossen ist.

Weitere Einzelheiten der Erfindung ergeben sich aus den Unteransprüchen. Die Erfindung ist anhand eines in den Figuren dargestellten Ausführungsbeispiels näher erläutert. Es zeigen:

Fig. 1 einen Längsschnitt durch eine Vorrichtung nach der Erfindung und

Fig. 2 einen Querschnitt durch die Vorrichtung nach Fig. 1 entlang der Linie II-II.

Die Fig. 1 zeigt eine Vorrichtung 1 zum Anwärmen und Befeuchten eines einem Patienten zugeführten Beatmungsgases in nicht maßstäblicher vergrößerter Darstellung. Die Vorrichtung 1 ist in einer Gasleitung 2 zwischen einem Patienten 3 und einem Beatmungsbehälter 4 angebracht. Die Vorrichtung 1 weist ein zylindrisches Gehäuse 5 auf, das an einem flanschartigen Stirnstück 6 mittels einer Bajonettenfassung 7 abnehmbar befestigt ist. Ein weiteres Stirnstück 8 ist über Halterungen 9, 10, 11 mit dem Gehäuse 5 fest verbunden. Zwischen den Stirnstücken 6 und 8 ist ein Hohlzylinder 12 aus hygroskopischem Material, der als Feuchtigkeitsspeicher dient, angeordnet.

Die Stirnseite 6, 8 und der Hohlzylinder 12 begrenzen
einen Hohlraum 13, der über die Gasleitung 2 mit dem
Patienten 3 verbunden ist. Das flanschartige Stirnstück 6 weist einen in den Hohlraum 13 ragenden kreuzartigen Teil 14 (Fig. 2) auf, dessen Außenmaß etwa dem
Innenmaß des Hohlzylinders 12 entspricht und dient als
Stütze für den Hohlzylinder 12. Zwischen der Wand des
Gehäuses 5 und dem Hohlzylinder 12 ist ein Spalt 17
vorgesehen, der zum gasbehälterseitigen Ende 18 des
Gehäuses 5 führt.

Der gasdurchlässige Hohlzylinder 12 besteht aus zwei
Schichten 15, 16. Das Material der inneren Schicht 16
hat eine höhere Gasdurchlässigkeit als das Material
der äußeren Schicht 15. Das Material der inneren
Schicht 16 kann z.B. Acrylfilz sein und das Material
der äußeren Schicht 15 kann z.B. Visco-Zellulose sein.
Die äußere Schicht 15 dient ausschließlich als Feuchtigkeitsspeicher, und die innere Schicht 16 als Feuchtigkeitsspeicher und als Wärmespeicher. Aufgrund der
verhältnismäßig großen Poren des Materials der inneren
Schicht 16 kann Feuchtigkeit und Wärme dort gespeichert
werden.

Beim Ausatmen des Patienten 3 strömt das Ausatmungsgas
durch die Gasleitung 2 in den Hohlraum 13 hinein und
staut sich zunächst dort. Dann strömt es durch die
Schichten 15, 16 des Hohlzylinders 12, der wie beschrieben einen großen Teil der Feuchtigkeit und Wärme des
Ausatmungsgases aufnimmt, hindurch und gelangt über
den Luftspalt 17 in ein Rohr 19, dessen freies Ende
in die Atmosphäre mündet. Beim Einatmen des Patienten 3
strömt trockenes Einatmungsgas vom Beatmungsgasbehälter 4 über die Gasleitung 2 in den Luftspalt 17 hinein und von dort durch den Hohlzylinder 12 hindurch. Das trockene Gas nimmt die im Hohlzylinder 12 gespeicherte Wärme und Feuchtigkeit auf, so daß das dem Patienten 3 zugeführte Beatmungsgas, das durch den Hohlraum 13 und die Gasleitung 2 strömt, die erwünschte Wärme und Feuchtigkeit beinhaltet.

Aufgrund der Tatsache, daß das Gehäuse 5 von dem flanschartigen Stirnstück 6 trennbar ist und daß das Stirnstück 8 am Gehäuse 5 befestigt ist, ist es einfach, den Hohlzylinder bei Bedarf auszuwechseln.

- 1 -    VPA 78 P 5951   EUR

Patentansprüche

1. Vorrichtung zum Anwärmen und Befeuchten eines Beatmungsgases für einen Patienten, die in einem Gehäuse, welches in einer zum Patienten und zu einem Beatmungsgasbehälter führenden Gasleitung angeordnet ist, einen Feuchtigkeitsspeicher aus gasdurchlässigem, hygroskopischem Material enthält, d a d u r c h  g e k e n n z e i c h n e t , daß in dem Gehäuse (5) ein mit der zum Patienten (3) führenden Gasleitung (2) verbundener Hohlraum (13) vorgesehen ist, der von einem Hohlzylinder (12) aus dem hygroskopischen Material umschlossen ist.

2. Vorrichtung nach Anspruch 1, d a d u r c h  g e k e n n z e i c h n e t, daß der Hohlraum (13) zwischen zwei Stirnstücken (6, 8) liegt, das eine Stirnstück (8) mit dem Gehäuse (5) unter Freilassung von Gasdurchlässen fest verbunden ist, und das andere Stirnstück an einer Gasleitung (2) für den Patienten befestigt ist und eine Öffnung für den Gasdurchtritt aufweist.

3. Vorrichtung nach Anspruch 2, d a d u r c h  g e k e n n z e i c h n e t , daß das Gehäuse (5) am anderen Stirnstück (6) abnehmbar befestigt ist.

4. Vorrichtung nach den Ansprüchen 1 bis 3, d a - d u r c h  g e k e n n z e i c h n e t , daß der Hohlzylinder (12) aus zwei Schichten (15, 16) besteht, bei denen das Material der inneren Schicht (16) größere Poren hat als das Material der äußeren Schicht (15).

0011847

- 2 -    VPA 78 P 5951 EUR

5. Vorrichtung nach Anspruch 4, d a d u r c h
g e k e n n z e i c h n e t , daß die äußere Schicht
(15) aus Visco-Zellulose und die innere Schicht (16)
aus Acrylfilz besteht.

0011847

78 P 5951    1/1

FIG 1

FIG 2

Europäisches
Patentamt

EUROPÄISCHER RECHERCHENBERICHT

0011847
Nummer der Anmeldung

EP 79 104 697.2

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE – A1 – 2 611 898 (TERUMO CORP.) <br> * Fig. 4 * <br> -- | 1 |
| | FR – A1 – 2 386 314 (SIEMENS AG) <br> * Ansprüche 1, 2; Fig. 1 * <br> -- | 1,3 |
| | US – A – 3 747 598 (K.W. COWANS) <br> * Ansprüche 1, 4; Fig. 2, 3, 6 * <br> -- | 1,2, 4 |
| A | DE – U – 7 723 608 (L. GIBECK) <br> * Ansprüche 1, 2 * <br> ---- | 1 |

### KLASSIFIKATION DER ANMELDUNG (Int.Cl.)

A 62 B    9/00
A 61 M    16/00

### RECHERCHIERTE SACHGEBIETE (Int. Cl.)

A 61 M    15/00
A 61 M    16/00
A 61 M    17/00
A 62 B    7/00
A 62 B    9/00

### KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X  Der vorliegende Recherchenbericht wurde für alle Patentansprüche erstellt.

| Recherchenort | Abschlußdatum der Recherche | Prüfer |
|---|---|---|
| Berlin | 28-02-1980 | KANAL |

EPA form 1503.1  06.78